# EUROPEAN PATENT SPECIFICATION

(11) **EP 2 932 905 B1**
(45) Date of publication and mention of the grant of the patent: **30.08.2017**
(21) Application number: 14164609.1
(22) Date of filing: 14.04.2014
(51) Int. Cl.: A61B 6/14, A61B 6/00, G03B 42/04

(54) **Case for X-ray plate, reading device and method of dental radiography**
Gehäuse für Röntgenplatte, Lesevorrichtung und Verfahren zur dentalen Radiographie
Boîtier pour plaque à rayons X, dispositif de lecture et procédé de radiographie dentaire

(43) Date of publication of application: 21.10.2015
(73) Proprietor: Nical S.p.A., 20125 Milano (IT)
(72) Inventor: Niccolucci, Roberto, I - 20124 MILAN (IT)
(74) Representative: Lunati & Mazzoni S.r.L.

(56) References cited:
- EP-A1- 0 219 821
- EP-A2- 0 115 802
- US-A1- 2002 036 756
- US-A1- 2005 205 813

## Description

The present invention relates to a case for an X-ray plate, a reading device and method of dental radiography of the type defined in the preamble of the independent claims.

Specifically, the invention relates to a device and method for reading a plate relative to an intraoral radiography.

Performing and reading an intraoral radiography involves the following operations. First, the physician encapsulates a photosensitive X-ray plate, for example a phosphor plate, in a case transparent to X-rays and opaque to light, so that the plate can only be exposed by the X-rays. In detail, in order to avoid undesirable exposure, the plate is fully housed in the case.

Next, the physician places the case in a sterile bag, arranges the plate-case-bag group in correspondence with the dental element to be X-rayed and takes the X-ray.

In the following step, the physician withdraws said group from the mouth, removes the sterile bag and arranges the plate and case in a reading device that extracts the plate from the case and thus performs the reading.

The reading device, using a magnet that is integral with the plate, extracts the X-ray plate from the case and places it in correspondence with a reading device.

In this step, a light beam stimulates the plate producing a quantity of photons proportional to the amount of X-ray energy received and which are collected by a suitable receiving element which, in turn, emits a signal proportional to the photons and used to reconstruct the X-ray image of the X-rayed dental element.

Esamples of cases for an X-ray plate are disclosed in US2005205813A1, EP0115802A2, EP0219821A1 and US2002036756A1.

The prior art mentioned above has some important drawbacks.

A first drawback is the complexity and, therefore, the slowness of the process owing to the extreme care with which the plate must be handled, in particular during its insertion into the bag.

Another important drawback regards the large dimensions of the prior art reading devices.

This aspect is particularly relevant in a private surgery, for example in a dental surgery, where there is a limited amount of space and so bulky equipment represents a considerable drawback.

A further drawback lies in the fact that when the reading device extracts the X-ray plate from the case, it may scratch and thus damage the plate.

A final, but no less important drawback lies in the cost of the X-ray plate which incorporates the magnet.

In this situation, the technical purpose of the present invention is to devise a case for an X-ray plate, a reading device and method of dental radiography capable of substantially overcoming the drawbacks mentioned above.

Within the scope of said technical purpose, an important aim of the invention is to provide a reading device and method of dental radiography that are fast and simple to use and implement.

Therefore, an important objective is to provide a case for an X-ray plate that facilitates these operations.

Another important aim of the invention is to provide a reading device that is compact in size, and that will not scratch or otherwise damage the plate.

The technical purpose and the specified aims are achieved with a case for an X-ray plate, a reading device and method of dental radiography as claimed in the appended independent claims.

Preferred embodiments are described in the dependent claims.

The characteristics and advantages of the invention are clearly evident from the following detailed description of a preferred embodiment of the invention, with reference to the accompanying drawings, in which:
**Fig. 1** shows a case according to the invention and an X-ray plate;
**Fig. 2** illustrates an X-ray plate inserted in the case of Fig. 1;
**Fig. 3** shows a top view of the reading device of dental radiography according to the invention in a first configuration;
**Fig. 4** shows a top view of the reading device of dental radiography according to the invention in a second configuration;
**Fig. 5** is a top view of the reading device of dental radiography according to the invention in a third configuration;
**Fig. 6** shows a side view of a portion of the reading device of dental radiography according to the invention in a first configuration;
**Fig. 7** schematically illustrates a side view of a portion of the reading device of dental radiography according to the invention in a second configuration; and

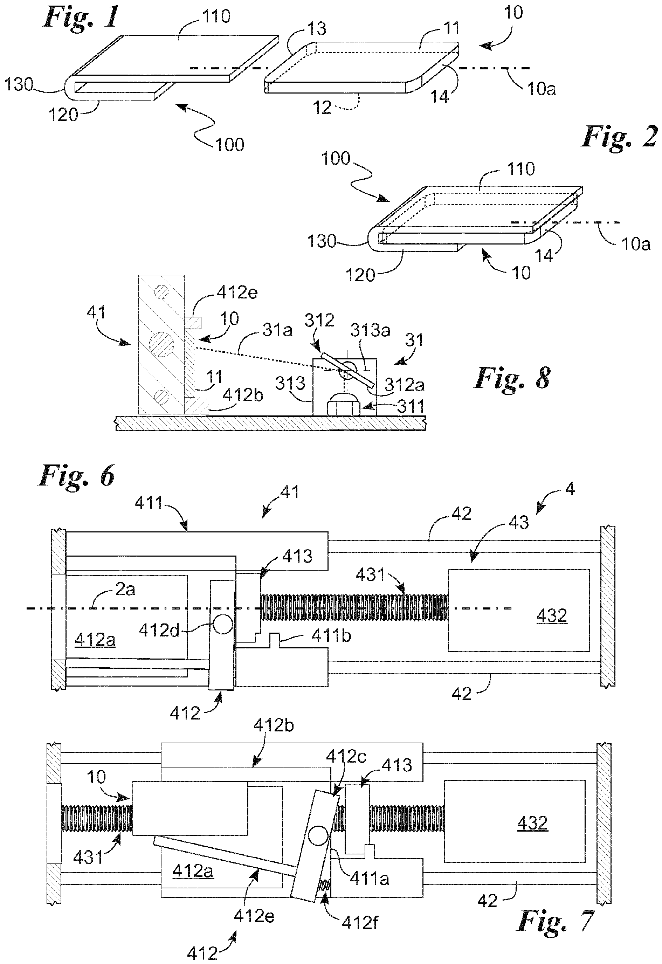

**Fig. 6** shows a section view of a further portion of the reading device of dental radiography according to the invention.

With reference to the above Figures, the reading device of dental radiography according to the invention is globally designed by number **1**.

It is suitable to be used for reading an intraoral radiography implemented on an X-ray plate **10** and, conveniently, a photostimulable phosphor plate 10, commonly called "imaging plate". It is also suitably devoid of magnetic elements for transport. The X-ray plate 10 has a surface **11** that is exposable by X-rays, preferably having phosphors that are photosensitive to said X-rays; a surface **12** that is not exposable by X-rays, opposite to the exposable surface 11; and a side surface around the surfaces 11 and 12.

Moreover, it is preferably substantially rectangular in shape so that the surfaces 11 and 12 have a short side and a long side.

The reading device 1 is suitable for use to read an X-ray plate 10 contained in an innovative case **100** for the plate 10. The case 100 and the plate 10 together and combined define a protected X-ray plate.

Advantageously, the case 100 has the same dimensions as the X-ray plate 10 and, more precisely a width substantially equal to that of the short side of the X-ray plate 10. Therefore, it is possible to have several cases 100 of different sizes and, in particular, of different widths, so as to have specific cases for each type of plate 10.

As illustrated in Figs. 1 and 2, it includes, exclusively, a first sheet **110** suitable to overlap substantially the whole of the exposable surface 11; a second sheet **120** suitable to overlap only a portion of the non-exposable surface 12 so as to leave one portion uncovered and therefore visible; and a joint edge **130** of the sheets 11 and 12 suitable to define a direction of extraction **100a** of the X-plate 10 from the case 100 that is substantially straight and, preferably, substantially parallel to the long side of the plate 10.

The term substantially indicates that the first sheet 110 covers the whole of the surface 11, possibly leaving only negligible amounts of the edges or other portions of the surfaces uncovered.

The joint edge 130 is suitable to come into contact with part of the side surface of the plate 10 defining, for the plate 10 when inserted into the case 100, a contact profile **13** with the joint edge 130 and a free profile **14**, i.e. a portion of the side surface not adjacent to the edge 130, advantageously opposite to the contact profile 13. Preferably, the contact profile 13 and the free profile 14 are substantially perpendicular to the axis of extraction 100a.

The first sheet 110 is practically flat and made of a material transparent to X-rays, so that the exposable surface 11 is exposable by X-rays. It is also scratch-resistant and, in detail, it has a lining that is suitable to come into contact with the surface 11 made of polytetrafluoroethylene (PTFE), known with the trademark Teflon^{®}.

The second sheet 120 is practically flat and made of a material opaque to visible light.

Preferably, the second sheet 120 is suitable to overlap a portion of the non-exposable surface 12 proximal to the contact profile of the plate 10, so that the visible portion of the surface 12 is adjacent to the free profile of said plate 10.

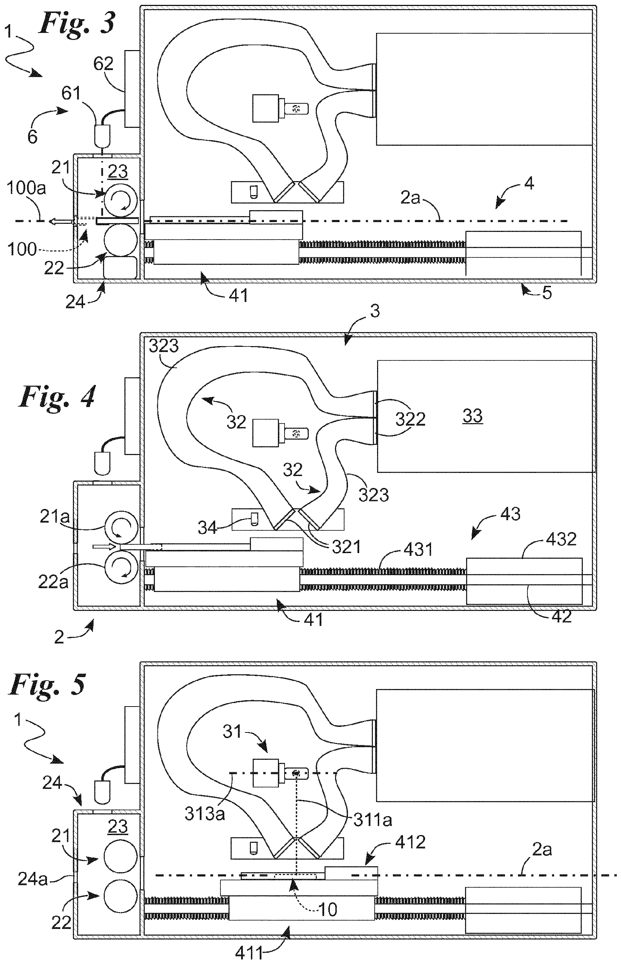

The reading device 1 (Figs. 3-5) includes, briefly, an extraction element **2** suitable to extract the plate 10 from the case 100; an X-ray unit suitable to perform the reading of the X-ray plate 10; and a power supply system, such as a battery and/or a connector to an external mains, suitable to electrically supply the X-ray unit and the extraction element 2.

The extraction element 2 comprises a first extractor **21** defining a first contact surface **21a** for the first sheet 110 and the exposable surface 11; a second extractor **22** defining a second contact surface **22a** for the non-exposable surface 12; a motor, conveniently an electric motor, suitable to reciprocally drive the extractors 21 and 22; a controller **23** suitable to control the extractors 21 and 22 so as to move the plate and/or the case along a sliding axis **2a**; and a crankcase **24** suitable to enclose therein the extractors 21 and 22 and defining an insertion slot **24a** in the element 2 for the case 100 and the plate 10.

The extractors 21 and 22 are mutually spaced so as to enclose between them the case 100 and the plate 10. In detail, they have contact surfaces **21a** and **22a** at a mutual distance that is practically equal to the thickness of the X-ray plate 10, so as to simultaneously come into contact with the first sheet 110 and the non-exposable surface 12. The distance between the rollers is also preferably variable by means of an elastic support of the rollers and/or the intrinsic elasticity of the rollers themselves.

Each extractor 21 and 22 comprises a roller, preferably made of elastomeric material, suitable to rotate about a practically transverse axis and, in particular, practically perpendicular to the direction 100a, and the side surface of which constitutes the contact surfaces 21 a and 22a.

The controller 23, as better described below, is suitable to control the extractors 21 and 22 so as to define a motion of extraction in which the plate 10 leaves the case thanks to a relative motion between the contact surfaces 21 a and 22a and, in particular, thanks to the different contact speeds of the surfaces 21 a and 22a respectively with the first sheet 110 and with the non-exposable surface 12, so that the case 100 is removed from the plate 10; and a forward motion in which it controls the forward motion of the plate 10 along the sliding axis 2a by moving the contact surfaces 21 a and 22a in such a way that the contact surfaces 21 a and 22a have speeds of contact, respectively, with the exposable surface 11 and with the non-exposable surface 12, substantially equal to one another, i.e. with speeds having the same direction and equal module, except for any negligible differences. The term contact speed indicates the speed and the direction of rotation (since the speed, as specified, comprises a module and a direction) of the contact surfaces 21 a and 22a at the point of contact with the plate 10 and/or the case 100. Specifically, in the case of extractors 21 and 22 consisting of rollers, the controller 23 moves the extractors at speeds of rotation in an opposite direction and substantially with the same module, so that said contact speeds are substantially equal to one another.

The X-ray unit comprises a reading element **3** suitable to perform the reading of the X-ray plate 10; a movement element **4** suitable to move the plate 10 between the extraction element 2 and the reading element 3; a control board suitable to control the operation of the X-ray unit; and a box-shaped member **5**, preferably a right-angled parallelepiped, suitable to contain the movement element 4 and reading element 3.

The movement element 4 (Figs. 6, 7) comprises a carriage **41** suitable to support the plate 10; at least one rail **42** defining an axis of movement substantially parallel to the sliding axis 2a, substantially parallel to the direction of extraction 100a, and a translation kinematic mechanism **43** capable of moving the carriage 41 along the rail 42. In detail, the movement element 4 comprises two rails 42 arranged symmetrically with respect to the translation kinematic mechanism 43.

The translation kinematic mechanism 43 comprises a worm **431** engaged with the carriage 41 and an axis substantially parallel to the sliding axis 2a; and a motor **432**, conveniently an electric motor, suitable to control the worm 431, and thus the carriage 41.

The carriage 41, as illustrated in Figs. 6 and 7 comprises a slide group **411** idly engaged with the rails 42; a constraint component **412** connected to the group 411 and suitable to secure the X-ray plate 10 to the slide group 411; and a slider **413** engaged with the worm 431 and suitable to control the slide group 411 and the constraint component 412.

The slider 413 controls the translation of the group 411 along the sliding axis 2a.

To this end, the slide group has a pressure surface **411a** on which the slider 413 presses when it brings the carriage 41 alongside the extraction element 2; and a protrusion **411b** against which the slider 413, moving in a direction opposite to the previous one, presses so as to draw the carriage 41 away from the extraction element 2.

The pressure surface 411 a and the protrusion 411 b are spaced apart along the sliding axis 2a so as to enclose the slider 413 between them and to allow said slider 413 to only come into contact with the surface 411 a or with the protrusion 411b. Preferably the distance, measured along the sliding axis 2a, between the protrusion 411 b and the pressure surface 411 a, is greater than the length of the slider 413 measured along the axis 2a.

The constraint element 412 comprises a backing plate **412a** for the non-exposable surface 12, integral with the group 411 and conveniently made of scratch-resistant material such as Teflon^{®}; a ledge **412b** for a face of the side surface, integral with the slide group 411 and practically parallel to the axis 2a; a lever **412c**, a hinge **412d** suitable to secure the lever 412c to the group 411; a detent **412e** integral with the lever 412c and suitable to press the plate 10 against the ledge 412b; and elastic means **412f**, preferably a compression spring, suitable to rotate the lever 412c so as to bring the detent 412e against the ledge 412b, and thus tighten the plate 10 between the detent 412e and the ledge 412b.

The detent 412e and the elastic means 412f are connected to the lever 412c, defining, with respect to the hinge 412d, a lever 412c of a second kind.

In order to allow the slider 413 to control the constraint element 412, the hinge 412d is proximal to the pressure surface 411 a and distant from the ends of the lever 412c, so that the end of the lever 412c distal from the detent 412e protrudes from the surface 411 a, allowing the cursor 413 to press the lever 412c when it comes in contact with said surface 411 a.

The reading element 3 comprises an emitter group **31** suitable to emit a laser beam **31a** or another light beam that, by striking the exposable surface 11, allows the plate 10 to produce blue photons; at least one collector **32** defining an intercepting surface **321** for said photons and a release surface **322** having an area almost equal to the surface 321 and through which the photons leave the collector 32; a reading unit **33** proximal to the release surface 322 so as to receive and analyse the photons; and a LED **34** or another similar element capable of erasing the image previously exposed on the X-ray plate 10 by the X-rays, allowing the same plate 10 to be used again.

Specifically, the reading element 3 comprises only one collector element 32 defining an intercepting surface 321, substantially parallel to the sliding axis 2a and, thus, to the exposable surface 11.

Alternatively, the reading element 3 has two collectors 32 with intercepting surfaces 321 preferably substantially perpendicular to each other and inclined at about 45° with respect to the sliding axis 2a and, thus, to the exposable surface 11. Preferably, the two collectors 32 have intercepting surfaces 321 suitably spaced so as to allow the laser beam 31a emitted by the emitter group 31 to pass between them and, thus, strike the exposable surface 12.

The emitter group 31 comprises a light source **311** suitable to emit the laser beam 31a and, specifically, a red laser beam suitable to produce a red light, namely red photons, striking the exposable surface 11; and an orienting mechanism suitable to direct the laser beam 31 a against the plate 10.

The source 311 is suitable to emit a laser light beam 31 a defining a propagation axis substantially perpendicular to the sliding axis 2a and substantially parallel to the exposable surface 11.

The orienting mechanism is suitable to deflect the laser beam 31 a against the surface 11.

As illustrated in Fig. 8, it comprises a reflective element **312** defining a mirroring surface **312a** for the laser beam 31a exiting the source 311; and a motor **313**, preferably an electric motor, suitable to rotate the first element 312 varying the angle of reflection and, thus, adjusting the point of incidence of the laser beam 31 a on the exposable surface 11.

The motor 313 is suitable to rotate the reflective element 312 about a rotation axis **313a** substantially parallel to the sliding axis 2a varying the output angle of the laser beam 31 a from the reflecting surface 312a and, thus, varying the point of incidence of the laser beam 31 a on the exposable surface 11 along a direction substantially perpendicular to the axis 2a.

In order to minimize the overall dimensions of the device 1, the release surface 322 is transverse and, in detail, substantially perpendicular to the intercepting surface 321, and the collector 32 comprises a transition block **323** suitable to transport the photons from the intercepting surface 321 to the release surface 322. The transition block 323 has a particular profile that allows the angle of incidence, i.e. the angle with respect to the perpendicular with the surface, between photons and the walls of the block 323, to be greater than the critical angle, i.e. than the minimum angle necessary to achieve the total reflection of the photons according to the known phenomenon exploited also in optical fibres, i.e. an angle enabling the internal reflection of the beam.

Moreover, to ensure a precise reconstruction of the images, each transition block 323 may be divided into a plurality of foils whose bases identify a portion of the surfaces 321 and 322, and having a body suitable to be crossed by the photons, thanks to the phenomenon of total reflection.

Preferably, the collector 32 is made in one piece and made of polymethylmethacrylate, PMMA, or other similar material transparent to blue light and, therefore, suitable to be crossed by the photons produced by the plate 10, without it suffer loss of energy.

Finally, the reading device 1 comprises a recognition element **6** suitable to identify the X-ray plate 10 and, thus, permit the optimal reading thereof.

Specifically, the recognition element 6 is suitable to identify the case 100 and, consequently, the X-ray plate 10 based on the width and, thus, the short side of the X-ray plate 10 or, preferably, of the case 100.

It is suitably arranged in correspondence with the extraction element 2 and, more precisely with the extractors 21 and 22 so as to identify the plate 10 before it arrives at the carriage 41. The recognition element 6 comprises a meter **61**, preferably an optical meter, suitable to measure the width of the case 100 and an identifier **62** suitable to identify the plate 10 according to the measurement of the meter 61 and to communicate the type of plate 10 to the reading element 3.

Specifically, the identifier 62 comprises a plate database which associates certain values of width of the case 100 with a specific plate 10 and, more specifically, the sizes of the long side and the short side of the X-ray plate 10 to be read.

The method of operation of the reading device of dental radiography, described above in structural sense, is as follows.

In detail, this method of operation defines an innovative method of reading preferably performed by means of the reading device 1.

The reading step is preceded by a step in which the physician prepares the X-ray plate 10; and an exposure step in which an X-ray is performed, preferably an intraoral X-ray.

In detail, during the preparation step, the plate 10 is placed in the case 100 making the first sheet 110 and the second sheet 120 overlap respectively the exposable surface 11 and a portion of the non-exposable surface 12, that remains partly uncovered. Then, the assembly comprising the plate 10 and the case 100 is inserted into a conventional sterile bag.

When the X-ray has been taken, the plate 10 and the case 100 are removed from the sterile bag and inserted through the insertion slot 24a into the device 1, so that the first sheet 110 is in contact with the first contact surface 21 a and the uncovered portion, proximal to the free profile 14 of the non-exposable surface 12, is in contact with the second surface 22a.

At this point the reading process begins and, specifically, an extraction step in which the X-ray plate 10 is extracted from the case 100.

In this step, Fig. 3, the controller 23 controls the extractors 21 and 22 to perform an extraction motion and, in particular, maintains the second extractor 22 practically stationary and rotates the first extractor 21 defining different contact speeds of the surfaces 21 a and 22a respectively with the first sheet 110 and with the non-exposable surface 12. These different contact speeds define a movement along the sliding axis 2a, of the case 100 only, which is thus separated from the plate 10 and ejected from the device 1 through the slot 24a.

It should be noted that at the end of the extraction step, the first contact surface 21 a is in contact with the exposable surface 11 and the second contact surface 22a is adjacent to the non-exposable surface 12.

Almost simultaneously with the extraction step, the reading process envisages an identification step in which the recognition element 6 identifies the X-ray plate 10. In this step, the meter 61 measures the width of the case 100 and, on the basis of said measurement, the identifier 62 identifies the plate 10 and communicates to the reading element 3 the type and, in particular, the size of the plate 10.

The reading process next envisages a step in which the X-ray plate 10 is moved.

In this step, Fig. 4, the controller 23 controls the extractors 21 and 22 to move forward by determining a contact speed of the surfaces 21 a and 22a with the surfaces 11 and 12, substantially equal to one another, so as to control the advancement of the plate 10 along the axis 2a, until the non-exposable surface 12 rests on the support plate 412a.

At this point, the translation kinematic mechanism 43 moves the slider 413 away from the pressure surface 411 a, by engaging it with the protrusion 411 b, so as to move the carriage 41 along the sliding axis 2a and lead the plate 10 to the reading element (Fig. 7).

Furthermore, the slider 413, moving away from the surface 411a, breaks away from the lever 41 c that, driven by the elastic means 412f, rotates tightening the X-ray plate 10 against the ledge 412b.

When the plate 10 reaches the collector 32, the movement step is completed and a scanning step starts, in which the reading element 3, based on the data collected by the recognition element 6, detects the image recorded on the X-ray plate 10.

In this step, while the carriage 41 moves forward, the light beam emitted by the emitter group 31, suitably deflected by the orienting mechanism, hits the exposable surface 11 exciting the X-ray plate 10 which, consequently, emits photons. Since the intercepting surfaces 321 are proximal to the exposable surface 11, the photons are intercepted and collected by the collector element 32 which carries them to the reading element 33.

Specifically, the laser beam 31 a exiting the source 312 strikes the mirroring surface 312a, which reflects and directs it against the exposable surface 11. Furthermore, the motor 313 rotates the reflective element 312 varying the inclination of the mirroring surface 312a. Such variation changes the output direction of the laser beam 31 a from it and, thus, the point of incidence of the laser beam on the exposable surface 11 along a direction almost perpendicular to the sliding axis 2a.

Furthermore, the simultaneous forward motion of the carriage 41 along the axis 2a varies, substantially along the sliding axis 2a, the point of incidence of the laser beam 31 a on the exposable surface 11, all of which is thus struck by the laser beam 31 a.

The data collected by the reading element 33 are transmitted via an appropriate connection, such as a cable or wireless connection, to a suitable mechanism that, interpreting the data, provides an image of the X-rayed body portion.

When the scanning step has been completed, the step in which the X-ray plate 10 is ejected from the reading device 1 is performed.

In this step, the slider 413, moving along the axis 2a in the opposite direction with respect to the movement step, presses on the pressure surface 411 a and, thus, controls the approach of the carriage 41 towards the extraction element 2.

Furthermore, the slider 413, when it comes into contact with the pressure surface 411 a, presses on the lever 412c that, consequently, rotates pushing the detent 412e away from the ledge 412b and, thus, removing the constraint of the X-ray plate 10 to the carriage 41 (Fig. 6).

When the carriage 41 reaches the extractors 21 and 22, the contact surfaces 21 a and 22a engage with the surfaces 11 and 12 and are moved simultaneously in the same direction extracting the plate 10 from the device 1 through the insertion slot 24a.

The invention achieves some important advantages.

A first advantage is the simplicity and speed of the reading process and, thus, the practicality of use of the reading device 1.

An important advantage lies in the fact that the reading device 1, when extracting the X-ray plate from the bag, does not incur the risk of scratching or otherwise damaging the exposable surface 11.

Such advantage is innovative due to the specific case 100 that, by lining the whole of the exposable surface 11 and part of the non-exposable surface 12, allows the contact surfaces 21 a and 22a to extract the X-ray plate 10 without coming into contact with the exposable surface 11.

Another advantage lies in the reduced dimensions that characterize the reading device 1.

Such aspect has been achieved thanks to the use of a collector 32 that, by having the release surface 322 transverse and, in detail, perpendicular to the intercepting surface 321, makes it possible to optimize the arrangement of the components and, therefore, to reduce the dimensions of the device 1.

A further advantage is the high quality of the X-ray image.

Such aspect has been achieved thanks to the use of two collectors 32 that essentially make it possible to double the amount of information available for the reconstruction of the X-ray image.

Modifications and variations may be made to the invention described herein without departing from the scope of the inventive concept. All elements described and claimed may be replaced by equivalent elements, and details, materials, shapes and dimensions may be of any type.

## Claims

1. Case (100) for X-ray plate (10); said X-ray plate (10) defining a surface (11) exposable by X-rays; a surface (12) not exposable by said X-rays, opposite to said exposable surface (11); said case (100) comprising
**characterized in that** said case (100) exclusively comprises
- a first sheet (110) suitable to overlap substantially all of said exposable surface (11)
- a second sheet (120) having an extension lower than the extension of said non-exposable surface (12) so that said second sheet (120) overlaps a portion of said non-exposable surface (12) defining a visible portion of said non-exposable surface (12);
- a joint edge (130) of said sheets (110, 120) defining an extraction direction (100a) of said X-ray plate (10) from said case (100);

2. Case (100) according to claim 1, wherein said X-ray plate (10) defines a side surface; wherein said joint edge (130) is suitable to come into contact with part of said side surface defining, for said X-ray plate (10) inserted in said case (100), a contact profile adjacent to said joint edge (130) and a free profile opposite said contact profile; and wherein said second sheet (120) is suitable to overlap a portion of said non-exposable surface (12) proximal to said contact profile, so that said visible portion is adjacent to said free profile.

3. Case (100) according to one or more of the preceding claims, wherein said first sheet (110) is transparent to said X-rays; wherein said second sheet (120) is opaque; and wherein said first sheet (110) is scratch-resistant and comprises a lining suitable to come into contact with said exposable surface (11) in polytetrafluoroethylene.

4. Protected X-ray plate, comprising an X-ray plate (10) devoid of magnetic means for transport, and a case (100) according to one or more of the preceding claims.

5. Reading device (1) for said X-ray plate (10) inserted in said case (100) according to one or more of claims 1-4 comprising
- an extraction element (2) suitable to extract said X-ray plate (10) from said case (100);
- a reading element (3) suitable to perform the reading of said X-ray plate (10);
- **characterized in that** said extraction element (2) comprises
- a first extractor (21) defining a first contact surface (21 a) for said first sheet (110);
- a second extractor (22) defining a second contact surface (22a) for said non-exposable surface (12);
- and a controller (23) suitable to control said extractors (21, 22) defining an extraction motion in which the contact speeds of said contact surfaces (21 a, 22a) differ from one another causing the extraction of said X-ray plate (10) from said case (100), and a forward motion in which the contact speeds of said contact surfaces (21 a, 22a) are the same as one another so as to move said X-ray plate (10) from said extraction element (2) to said reading element (3).

6. Reading device (1) according to the preceding claim, wherein each of said extractors (21, 22) comprises a roller suitable to rotate about an axis that is practically perpendicular to said extraction direction (100a).

7. Reading device (1) according to one or more of claims 5-6, wherein said reading element (3) comprises an emitter group (31) suitable to emit a light beam that, when it hits said exposable surface (11), produces photons; at least one collector (32) defining an intercepting surface (321) suitable to be arranged proximal to said exposable surface (11) and through which said photons enter therein, and a release surface (322) through which said photons leave said collector (32); and a reading element (33) proximal to said release surface (322) and suitable to receive said photons.

8. Reading device (1) according to claim 7, wherein said release surface (322) is transverse to said intercepting surface (321).

9. Reading device (1) according to one or more of claims 7-8, wherein said reading element (3) comprises two collectors (32); and wherein said intercepting surfaces of said collectors (32) are transverse with respect to one other.

10. Reading device (1) according to one or more of claims 7-9, wherein said at least one collector (32) is made of polymethylmethacrylate.

11. Reading device (1) according to one or more of the preceding claims, comprising a recognition element (6) suitable to identify said X-ray plate (10) based on the width of said case (100).

12. Method of reading said X-ray plate (10) inserted in said case (100) according to one or more of claims 1-4, comprising
- an extraction step in which an extraction element (2) extracts said X-ray plate (10) from said case (100);
- a scanning step in which a reading element (3) detects the image recorded on said X-ray plate (10);
- a movement step in which said X-ray plate (10) is moved from said extraction element (2) to said reading element (3);
- **characterized in that** in said extraction step
- said X-ray plate (10) and said case (100) are brought into contact with a first contact surface (21 a) and a second contact surface (22a) placing said first sheet (110) in contact with said first contact surface (21 a) and said non-exposable surface (12) in contact with said second contact surface (22a); and
- said first contact surface (21 a) and said second contact surface (22a) are mutually moved with different contact speeds so as to extract said X-ray plate (10) from said case (100) and bring said first contact surface (21 a) in contact with said exposable surface (11);
- and **in that** in said movement step
- said first contact surface (21 a) and said second contact surface (22a) are moved by defining speeds of contact with said surfaces (11, 12) substantially equal to each other and, consequently, moving said X-ray plate (10).

## Patentansprüche

1. Gehäuse (100) für Röntgenplatte (10); wobei die Röntgenplatte (10) eine röntgenstrahlenempfindliche Oberfläche (11) definiert; eine nicht für die genannten Röntgenstrahlen empfindliche, der genannten röntgenstrahlenempfindlichen Oberfläche (11) gegenüberliegende Oberfläche (12);
- **dadurch gekennzeichnet, dass** das genannte Gehäuse (100) ausschließlich Folgendes umfasst:
- ein erstes Blatt (110), das geeignet ist, im Wesentlichen die Gesamtheit der genannten röntgenstrahlenempfindlichen Oberfläche (11) abzudecken
- ein zweites Blatt (120) mit einer geringeren Ausdehnung im Vergleich zur Ausdehnung der genannten nicht röntgenstrahlenempfindlichen Oberfläche (12), so dass das genannte zweite Blatt (120) einen Abschnitt der genannten nicht röntgenstrahlenempfindlichen Oberfläche (12) abdeckt und so einen sichtbaren Abschnitt der genannten nicht röntgenstrahlenempfindlichen Oberfläche (12) definiert; und
- einen Rand zur Verbindung (130) der genannten Blätter (110, 120), der eine Entnahmerichtung (100a) der genannten Röntgenplatte (10) aus dem genannten Gehäuse (100) definiert.

2. Gehäuse (100) nach Anspruch 1, bei dem die genannte Röntgenplatte (10) eine Seitenfläche definiert; bei dem der genannte Rand zur Verbindung (130) geeignet ist, mit einem Teil der genannten Seitenfläche in Kontakt zu kommen und für die in das genannte Gehäuse (100) eingesetzte Röntgenplatte (10) ein an den genannten Rand zur Verbindung (130) angrenzendes Kontaktprofil und ein dem genannten Kontaktprofil gegenüberliegendes freies Profil zu definieren; und bei dem das genannte zweite Blatt (120) geeignet ist, einen Abschnitt der genannten nicht röntgenstrahlenempfindlichen Oberfläche (12) proximal zu dem genannten Kontaktprofil abzudecken, so dass der genannte sichtbare Abschnitt an das genannte freie Profil angrenzt.

3. Gehäuse (100) nach einem oder mehreren der vorangegangenen Ansprüche, bei dem das genannte erste Blatt (110) für die genannten Röntgenstrahlen transparent ist; bei dem das zweite Blatt (120) matt ist; und bei dem das genannte erste Blatt (110) kratzfest ist und eine Beschichtung umfasst, die geeignet ist, mit der genannten röntgenstrahlenempfindlichen Oberfläche (11) aus Polytetrafluorethylen in Kontakt zu kommen.

4. Geschützte Röntgenplatte, umfassend eine Röntgenplatte (10) ohne Magnetelemente für den Transport und ein Gehäuse (100) nach einem oder mehreren der vorangegangenen Ansprüche.

5. Lesevorrichtung (1) für die genannte in das genannte Gehäuse (100) eingesetzte Röntgenplatte (10) nach einem oder mehreren der Ansprüche 1 - 4, die Folgendes umfasst:
- ein Entnahmeorgan (2), das geeignet ist, die genannte Röntgenplatte (10) aus dem genannten Gehäuse (100) zu entnehmen;
- ein Leseorgan (3), das geeignet ist, das Ablesen der genannten Röntgenplatte (10) auszuführen;
- **dadurch gekennzeichnet, dass** das genannte Entnahmeorgan (2) Folgendes umfasst:
- eine erste Entnahmevorrichtung (21), die eine erste Kontaktfläche (21 a) für das genannte erste Blatt (110) definiert;
- eine zweite Entnahmevorrichtung (22), die eine zweite Kontaktfläche (22a) für die genannte nicht röntgenstrahlenempfindliche Oberfläche (12) definiert;
- und einen Regler (23), der geeignet ist, die genannten Entnahmevorrichtungen (21, 22) zu steuern, indem er eine Entnahmebewegung, bei der die Kontaktgeschwindigkeiten der genannten Kontaktflächen (21 a, 22a) voneinander verschieden sind und das Entnahmen der genannten Röntgenplatte (10) aus dem genannten Gehäuse (100) bewirken, und eine Vorschubbewegung, bei der die Kontaktgeschwindigkeiten der genannten Kontaktflächen (21a, 22a) gleich sind, definiert, so dass die genannte Röntgenplatte (10) von dem genannten Entnahmeorgan (2) zu dem genannten Leseorgan (3) bewegt wird.

6. Lesevorrichtung (1) nach dem vorangegangenen Anspruch, bei der jede der genannten Entnahmevorrichtungen (21, 22) eine Rolle umfasst, die in der Lage ist, um eine zu der genannten Entnahmerichtung (100a) nahezu normale Achse zu drehen.

7. Lesevorrichtung (1) nach einem oder mehreren der Ansprüche 5 - 6, bei der das genannte Leseorgan (3) eine Sendergruppe (31) umfasst, die geeignet ist, ein Strahlenbündel auszusenden, das durch Gravieren der röntgenstrahlenempfindlichen Oberfläche (11) Photonen produziert; wenigstens einen Aufnahmevorrichtung (32), die eine Auffangfläche (321), die geeignet ist, proximal zu der genannten röntgenstrahlenempfindlichen Oberfläche (11) angeordnet zu werden und über die die genannten Photonen in ihr Inneres gelangen, und eine Abgabefläche (322), über die die Photonen die genannte Aufnahmevorrichtung (32) verlassen, definiert; und eine Leseeinheit (33) proximal zu der genannten Abgabefläche (322), die geeignet ist, die genannten Photonen aufzunehmen.

8. Lesevorrichtung (1) nach Anspruch 7, bei der die genannte Abgabefläche (322) quer zu der genannten Auffangfläche (321) liegt.

9. Lesevorrichtung (1) nach einem oder mehreren der Ansprüche 7 - 8, bei der das genannte Leseorgan (3) zwei Aufnahmevorrichtungen (32) umfasst; und bei der die genannten Auffangflächen der genannten Aufnahmevorrichtungen (32) zueinander quer liegen.

10. Lesevorrichtung (1) nach einem oder mehreren der vorangegangenen Ansprüche 7-9, bei der mindestens eine Aufnahmevorrichtung (32) aus Polymethylmethacrylat besteht.

11. Lesevorrichtung (1) nach einem oder mehreren der vorangegangenen Ansprüche, umfassend ein Erkennungsorgan (6), das in der Lage ist, die genannte Röntgenplatte (10) abhängig von der Breite des genannten Gehäuses (100) zu erkennen.

12. Verfahren zum Lesen der genannten, in das genannte Gehäuse (100) eingesetzten Röntgenplatte (10) nach einem oder mehreren der Ansprüche 1 - 4, das Folgendes umfasst:
- einen Entnahmeschritt, bei dem ein Entnahmeorgan (2) die genannte Röntgenplatte (10) aus dem genannten Gehäuse (100) entnimmt;
- einen Abtastschritt, bei dem ein Leseorgan (3) das auf der genannten Röntgenplatte (10) abgedruckte Bild erfasst;
- einen Bewegungsschritt, bei dem die genannte Röntgenplatte (10) von dem genannten Entnahmeorgan (2) zu dem genannten Leseorgan (3) bewegt wird;
- **dadurch gekennzeichnet, dass** bei diesem Entnahmeschritt
- die genannte Röntgenplatte (10) und das genannte Gehäuse (100) mit einer ersten Kontaktfläche (21 a) und einer zweiten Kontaktfläche (22a) in Kontakt gebracht werden, so dass das genannte erste Blatt (110) in Kontakt mit der genannten ersten Kontaktfläche (21 a) und die genannte nicht röntgenstrahlenempfindliche Oberfläche (12) in Kontakt mit der genannten zweiten Kontaktfläche (22a) gebracht wird; und
- die genannte erste Kontaktfläche (21 a) und die genannte zweite Kontaktfläche (22a) jeweils mit unterschiedlichen Kontaktgeschwindigkeiten bewegt werden, so dass die genannte Röntgenplatte (10) aus dem genannten Gehäuse (100) entnommen und die genannte erste Kontaktfläche (21 a) in Kontakt mit der genannten röntgenstrahlenempfindlichen Oberfläche (11) gebracht wird;
- und dadurch, dass bei diesem Bewegungsschritt
- die genannte erste Kontaktfläche (21 a) und die genannte zweite Kontaktfläche (22a) bewegt werden und Kontaktgeschwindigkeiten mit den genannten Oberflächen (11, 12) definieren, die im Wesentlichen gleich sind und folglich die genannte Röntgenplatte (10) bewegen.

## Revendications

1. Boîtier (100) pour plaque à rayons X (10) ; ladite plaque à rayons X (10) définissant une surface pouvant être exposée (11) à des rayons X ; une surface ne pouvant pas être exposée (12) auxdits rayons X opposée à ladite surface pouvant être exposée (11) ;
- **caractérisé en ce que** ledit boîtier (100) comprend exclusivement
- une première feuille (110) destinée à se superposer essentiellement à la totalité de ladite surface pouvant être exposée (11)
- une seconde feuille (120) ayant une extension inférieure par rapport à l'extension de ladite surface ne pouvant pas être exposée (12) de manière à ce que ladite seconde feuille (120) se superpose à une portion de ladite surface ne pouvant pas être exposée (12) définissant une portion visible de ladite surface ne pouvant pas être exposée (12) ; et
- un bord de jonction (130) desdites feuilles (110, 120) définissant une direction d'extraction (100a) de ladite plaque à rayons X (10) dudit boîtier (100).

2. Boîtier (100) selon la revendication 1, où ladite plaque à rayons X (10) définit une surface latérale ; où ledit bord de jonction (130) est destiné à être mis au contact d'une partie de ladite surface latérale définissant, pour ladite plaque à rayons X (10) introduite dans ledit boîtier (100), un profil de contact adjacent audit bord de jonction (130) et un profil libre opposé audit profil de contact ; et où ladite seconde feuille (120) est destinée à se superposer à une portion de ladite surface ne pouvant pas être exposée (12) à proximité dudit profil de contact de manière à ce que ladite portion visible soit adjacente audit profil libre.

3. Boîtier (100) selon une ou plusieurs des revendications précédentes, où ladite première feuille (110) est transparente auxdits rayons X ; où ladite seconde feuille (120) est opaque; et où ladite première feuille (110) est antirayures et comprend un revêtement destiné à être mis au contact de ladite surface pouvant être exposée (11) en polytétrafluoroéthylène.

4. Plaque à rayons X protégée, comprenant une plaque à rayons X (10) dépourvue de moyens magnétiques pour le transport et un boîtier (100) selon une ou plusieurs des revendications précédentes.

5. Dispositif de lecture (1) pour ladite plaque à rayons X (10) introduite dans ledit boîtier (100) selon une ou plusieurs des revendications 1-4 comprenant
- un organe d'extraction (2) destiné à extraire ladite plaque à rayons X (10) dudit boîtier (100) ;
- un organe de lecture (3) destiné à exécuter la lecture de ladite plaque à rayons X (10) ;
- **caractérisé en ce que** ledit organe d'extraction (2) comprend
- un premier extracteur (21) définissant une première surface de contact (21 a) pour ladite première feuille (110) ;
- un second extracteur (22) définissant une seconde surface de contact (22a) pour ladite surface ne pouvant pas être exposée (12) ;
- et un contrôleur (23) destiné à commander lesdits extracteurs (21, 22) définissant un mouvement d'extraction où les vitesses de contact desdites surfaces de contact (21 a, 22a) sont différentes l'une de l'autre, provoquant l'extraction de ladite plaque à rayons X (10) dudit boîtier (100) et un mouvement d'avance où les vitesses de contact desdites surfaces de contact (21a, 22a) sont égales l'une à l'autre de manière à déplacer ladite plaque à rayons X (10) dudit organe d'extraction (2) audit organe de lecture (3).

6. Dispositif de lecture (1) selon la revendication précédente, où chacun desdits extracteurs (21, 22) comprend un rouleau destiné à tourner autour d'un axe presque normal dans ladite direction d'extraction (100a).

7. Dispositif de lecture (1) selon une ou plusieurs des revendications 5-6, où ledit organe de lecture (3) comprend un groupe émetteur (31) destiné à émettre un faisceau lumineux qui, en gravant ladite surface pouvant être exposée (11), produit des photons ; au moins un collecteur (32) définissant une surface d'interception (321) destinée à être disposée à proximité de ladite surface pouvant être exposée (11) et à travers laquelle lesdits photons entrent à l'intérieur dudit collecteur et une surface de rejet (322) à travers laquelle lesdits photons abandonnent ledit collecteur (32) ; et une unité de lecture (33) à proximité de ladite surface de rejet (322) et destinée à recevoir lesdits photons.

8. Dispositif de lecture (1) selon la revendication 7, où ladite surface de rejet (322) est transversale à ladite surface d'interception (321).

9. Dispositif de lecture (1) selon une ou plusieurs des revendications 7-8, où ledit organe de lecture (3) comprend deux collecteurs (32) ; et où lesdites surfaces d'interception desdits collecteurs (32) sont transversales l'une par rapport à l'autre.

10. Dispositif de lecture (1) selon une ou plusieurs des revendications 7-9, où au moins ledit collecteur (32) est en polyméthacrylate de méthyle.

11. Dispositif de lecture (1) selon une ou plusieurs des revendications précédentes, comprenant un organe de reconnaissance (6) destiné à identifier ladite plaque à rayons X (10) en fonction de la largeur dudit boîtier (100).

12. Procédé de lecture de ladite plaque à rayons X (10) introduite dans ledit boîtier (100) selon une ou plusieurs des revendications 1-4, comprenant
- une phase d'extraction où un organe d'extraction (2) extrait ladite plaque à rayons X (10) dudit boîtier (100) ;
- une phase de balayage où un organe de lecture (3) relève l'image imprimée sur ladite plaque à rayons X (10) ;
- une phase d'actionnement où ladite plaque à rayons X (10) est déplacée dudit organe d'extraction (2) audit organe de lecture (3) ;
- **caractérisé en ce que** pendant ladite phase d'extraction
- ladite plaque à rayons X (10) et ledit boîtier (100) sont placés au contact d'une première surface de contact (21 a) et d'une seconde surface de contact (22a) en disposant ladite première feuille (110) au contact de ladite première surface de contact (21 a) et ladite surface ne pouvant pas être exposée (12) au contact de ladite seconde surface de contact (22a) ; et
- ladite première surface de contact (21 a) et ladite seconde surface de contact (22a) sont réciproquement actionnées à des vitesses de contact différentes l'une de l'autre de manière à extraire ladite plaque à rayons X (10) dudit boîtier (100) et à amener ladite première surface de contact (21a) au contact de ladite surface pouvant être exposée (11) ;
- et par le fait que durant ladite phase d'actionnement
- ladite première surface de contact (21 a) et ladite seconde surface de contact (22a) sont actionnées en définissant des vitesses de contact avec lesdites surfaces (11, 12) essentiellement égales l'une à l'autre et, en conséquence, en déplaçant ladite plaque à rayons X (10).
